# EUROPEAN PATENT SPECIFICATION

(11) **EP 3 177 154 B1**
(45) Date of publication and mention of the grant of the patent: **25.09.2019**
(21) Application number: 15750350.9
(22) Date of filing: 06.08.2015
(51) Int. Cl.: A23L 33/135, A23L 33/10, A61K 35/745, A61K 35/747, A61K 31/047

(54) **MYO-INOSITOL AND PROBIOTICS, AND THEIR USE TO PREVENT EXCESS BODY WEIGHT IN INFANTS**
MYO-INOSITOL UND PROBIOTIKA, UND IHRE VERWENDUNG ZUR VERHINDERUNG VON ÜBERGEWICHT BEI SÄUGLINGEN
MYO-INOSITOL ET PROBIOTIQUES ET LEUR UTILISATION POUR LA PREVENTION DE L'EXCES DE POIDS DES NOURRISSONS

(30) Priority: 08.08.2014 EP 14180403; 05.06.2015 EP 15170915
(43) Date of publication of application: 14.06.2017
(73) Proprietor: Société des Produits Nestlé S.A., 1800 Vevey (CH)
(72) Inventor: SILVA ZOLEZZI, Irma, CH-1084 Carouge (CH); MACE, Catherine, CH-1005 Lausanne (CH); BUDIN, Florence, CH-1110 Morges (CH); GODFREY, Keith Malcolm, Ashurst Hampshire SO40 7AP (GB); BAKER, Philip Newton, Auckland 1011 (NZ); CHONG, Yap Seng, Singapore 268431 (SG)
(74) Representative: Krishnan, Sri
(86) International application number: PCT/EP2015/068192
(87) International publication number: WO 2016/020491

(56) References cited:
- EP-A1- 2 011 506
- EP-A1- 2 452 575
- US-A- 5 763 392
- US-A1- 2004 072 794
- US-A1- 2010 111 915
- KIRSI LAITINEN ET AL: "Probiotics and dietary counselling contribute to glucose regulation during and after pregnancy: a randomised controlled trial", BRITISH JOURNAL OF NUTRITION, vol. 101, no. 11, 19 November 2008 (2008-11-19), page 1679, XP055164118, ISSN: 0007-1145, DOI: 10.1017/S0007114508111461
- RAAKEL LUOTO ET AL: "Impact of maternal probiotic-supplemented dietary counselling on pregnancy outcome and prenatal and postnatal growth: a double-blind, placebo-controlled study", BRITISH JOURNAL OF NUTRITION, vol. 103, no. 12, 4 February 2010 (2010-02-04), pages 1792-1799, XP55030021, ISSN: 0007-1145, DOI: 10.1017/S0007114509993898
- MARINE L. CROZE ET AL: "Chronic treatment with myo-inositol reduces white adipose tissue accretion and improves insulin sensitivity in female mice", THE JOURNAL OF NUTRITIONAL BIOCHEMISTRY, vol. 24, no. 2, 1 February 2013 (2013-02-01), pages 457-466, XP055212044, ISSN: 0955-2863, DOI: 10.1016/j.jnutbio.2012.01.008

## Description

### Technical field

The invention relates to myo-inositol and probiotics and their use to improve the body composition of an infant, in particular to prevent excess weight and/or to increase the % lean mass of an infant.

### Background

A sub-optimal body composition in infancy e.g. an excess body weight and/or a lower % of lean body mass, may have long term effects on the health and wellbeing of a subject e.g. a mammal such as a human, cat or dog. In particular, a sub-optimal body composition in infancy, more particularly excess weight and/or a lower % of lean body weight, may be associated with an elevated weight status in later life, which in turn may be a risk factor for a variety of immediate and long term conditions e.g. Type II diabetes, cardio vascular disease, and stroke.

Unfortunately, the number of infants having a sub-optimal body composition, in particular infants having excess weight and/or a lower % of lean body mass, is increasing globally. Because of the increasing prevalence, and because of the associated health and wellness concerns, addressing this problem is seen as a key public health challenge.

Accordingly there is a need to find ways to improve body composition in infancy, in particularly there is a need to find ways to prevent the development of excess weight in infants and/or to improve the % of lean body mass in infants. The use of probiotics for reducing the risk of development of overweight or obesity of an infant later in life is disclosed in US 2010/0111915. Croze et al, J. Nutr. Biochem. 2013; 24: 457-466, reported a preventive role of a chronic treatment with myo-inositol in white adipose tissue accretion, and an improvement in insulin sensitivity, in female mice.

Surprisingly the inventors have found that the in-utero administration of a combination of myo-inositol and probiotics may result in an improved body composition in an infant, in particular the inventors have found that the in-utero administration of a combination of myo-inositol and probiotics may result in a lower body weight and a higher % of lean mass in an infant.

There is an increasing body of evidence suggesting that the intrauterine environment may play a key role in determining the body composition of an infant, and it is thought that the altering of the intrauterine environment can affect the offspring physiology including body composition of said offspring. However, many factors can affect the intrauterine environment and it is not known which specific factors are significant for this effect.

### Summary of the Invention

The invention is set out in the claims, and relates to myo-inositol and probiotics for use to prevent excess body weight of an infant, wherein said myo-inositol and probiotics are administered simultaneously, separately or sequentially to said infant in utero and optionally also after birth wherein, said probiotics comprise a combination of Lactobacillus and Bifidobacterium, and wherein the Lactobacillus is the Lactobacillus rhamnosus GG strain available under the deposit number CGMCC 1.3724, and the Bifidobacterium is the Bifidobacterium lactis BB12 strain deposited as CNCMI-3446. The inventors have found that the in-utero administration of a combination of myo-inositol and probiotics may result in a lower body weight and higher % lean mass in an infant. Accordingly, myo-inositol and probiotics may be used to improve the body composition of an infant, in particular to prevent excess weight and/or to increase the % lean mass in an infant.

Said myo-inositol and probiotics may be administered simultaneously, separately or sequentially to said infant in-utero. Said administration may be via the pregnant mother. Said administration may optionally continue after birth. Administration to the infant after birth may be directly to the infant and/or via the mother in said mother's breast milk. Administration after birth may for example continue until the infant is 12 months of age.

The probiotic of the invention comprises a combination of Lactobacillus and Bifidobacterium, in particular the Lactobacillus is the Lactobacillus rhamnosus GG strain available under the deposit number CGMCC 1.3724, and/or the Bifidobacterium is the Bifidobacterium lactis BB12 strain deposited as CNCM 1-3446.

If the myo-inositol and probiotics are administered in-utero and optionally after birth via said mother's breast milk, they may be administered in one or more composition suitable for maternal administration, for example they may be administered in the form of a powdered nutritional composition, a food product, a functional food product, a drink (beverage), a dairy product, a pharmaceutical formulation, a pet food product, a nutraceutical, a nutritional supplement e.g. a powdered nutritional supplement e.g. to be sprinkled on food or dissolved in an aqueous medium e.g. water, juice, or milk.

If after birth the myo-inositol and probiotics are administered directly to the infant they may be administered in one or more composition suitable for consumption by an infant e.g. in the form of an infant formula, a composition for infants that is intended to be added or diluted with HM e.g. HM fortifier, and/or food stuffs intended for consumption by infants either alone or in combination with HM.

It may be advantageous if the myo-inositol and probiotics are simultaneously, separately or sequentially administered with one or more of vitamin B2, vitamin B12, vitamin B6, and vitamin D. Said vitamins have a host of beneficial health effects associated with them, and in particular it is thought that maternal administration of one or more of these vitamins may affect the adiposity, or risk of obesity in an infant.

The myo-inositol and probiotics may be administered in any effective dose. However, particularly effective doses may be 0.2 to 5 mg of myo-inositol, 10⁵ to 10¹² cfu of probiotics. Particularly effective doses of vitamin B2, vitamin B6, vitamin B12 and vitamin D may be 0.14 to 14 mg of vitamin B2, 0.19 to 19 mg of vitamin B6, 0.26 to 26 µg of vitamin B12, 1.5 to 100 µg of vitamin D.

The myo-inositol and probiotics may be provided in a kit for use to improve the body composition of an infant, more particularly to prevent excess weight and/or to increase the % lean mass of an infant.

The myo-inositol and probiotics may also be used in the manufacture of a composition for use to improve the body composition of an infant, in particular to prevent excess weight in an infant or to increase the % lean body mass of an infant.

### Detailed Description

In a first aspect of the present invention there is provided myo-inositol and probiotics, which probiotics comprise a combination of Lactobacillus rhamnosus GG strain available under the deposit number CGMCC 1.3724, and Bifidobacterium lactis BB12 strain deposited as CNCM 1-3446, for use to to prevent excess body weight of an infant, wherein said myo-inositol and probiotics are administered simultaneously, separately or sequentially to said infant in-utero and optionally also after birth. The term "infant" as used herein refers to a mammal infant, in particular a human infant, and more particularly a human infant of 12 months of age or less.

The term "prevent" as used herein refers to the prevention of the occurrence, or reduction of the risk of the occurrence, of a condition e.g. excess weight in a subject.

The term myo-inositol as used herein refers to myo-inositol (cis-1,2,3,5-trans-4,6-cyclohexanehexol) and /or a metabolite thereof.

Any source of myo-inositol suitable for ingestion by the pregnant subject may be used in the invention.

A metabolite of myo-inositol can be selected from the group consisting of D-chiro-inositol, L-chiro-inositol and a combination of the foregoing. In particular the metabolite is D-chiro-inositol.

The term probiotic as used herein refers to live probiotic bacteria, non-replicating probiotic bacteria, dead probiotic bacteria, non-viable probiotic bacteria, fragments of probiotic bacteria such as DNA, metabolites of probiotic bacteria, cytoplasmic compounds of probiotic bacteria, cell wall materials of probiotic bacteria, culture supernatants of probiotic bacteria, and combinations of any of the foregoing.

In particular the probiotic is live probiotic bacteria non-replicating probiotic bacteria, dead probiotic bacteria, non-viable probiotic bacteria, and any combination thereof. More particularly the probiotic is live probiotic bacteria.

The probiotics comprise Lactobacillus and/or Bifidobacterium, wherein the Lactobacillus strain is the Lactobacillus rhamnosus GG strain available under the deposit number CGMCC 1.3724, and the Bifidobacterium strain is the Bifidobacterium lactis BB12 strain deposited as CNCM 1-3446. More particularly the probiotics comprises a mixture of the Lactobacillus rhamnosus GG strain deposited by Nestle R&D centre Shanghai Ltd (13 Qiao Nan, Cao An Road, Jiading District, Shanghai 201812, P.R. China) at the China General Microbiological Culture Collection Centre (CGMCC) and available under the deposit number CGMCC 1.3724 and of the Bifidobacterium lactis BB12 strain deposited at the Collection Nationale de Cultures De Microorganismes (CNCM) as CNCM 1-3446. Even more particularly the probiotics consist of a mixture of the Lactobacillus rhamnosus GG strain available under the deposit number CGMCC 1.3724 and of the Bifidobacterium lactis BB12 strain deposited as CNCM 1-3446.

In an embodiment the myo-inositol is cis-1,2,3,5-trans-4,6-cyclohexanehexol and the probiotic comprises or consists of a mixture of the Lactobacillus rhamnosus GG strain available under the deposit number CGMCC 1.3724 and of the Bifidobacterium lactis BB12 strain deposited as CNCM 1-3446.

The myo-inositol and probiotics may be provided in a format providing sustained release of said vitamins or one or more probiotic. This way, these compounds can be consumed less frequently, while the body is still constantly supplied with a sufficient amount of them.

The myo-inositol and probiotics may be administered in-utero by any known means.

In an embodiment the myo-inositol and probiotics are administered via the pregnant mother.

The myo-inositol and probiotics may be administered after birth to an infant by any known means e.g. through the mother via the mother's breast milk or directly to the infant e.g. in infant formula.

In an embodiment the myo-inositol and probiotics are administered to the infant via the mother's breast milk.

Administration of the myo-inositol and probiotics to the infant may continue for any length of time after birth, for example administration may continue until the infant is 1, 2, 3, 4, 5, 6, 7, 8, 9, 10, 11, or 12 months of age.

In an embodiment administration to the infant continues after birth until the infant is 12 months of age.

If the myo-inositol and probiotics are administered to an infant in-utero via the pregnant mother, and optionally after birth via the mother's breast milk, they may be administered to said pregnant mother or mother in one or more composition suitable for maternal administration, for example they may be administered in the form of a powdered nutritional composition, a food product, a functional food product, a drink (beverage), a dairy product, a pharmaceutical formulation, a pet food product, a nutraceutical, a nutritional supplement e.g. a powdered nutritional supplement e.g. to be sprinkled on food or dissolved in an aqueous medium e.g. water, juice, or milk.

If after birth the myo-inositol and probiotics are administered directly to the infant they may be administered in one or more composition suitable for consumption by an infant e.g. in the form of an infant formula, a composition for infants that is intended to be added or diluted with HM e.g. HM fortifier, and/or food stuffs intended for consumption by infants either alone or in combination with HM.

The term "food product", as used herein, refers to any kind of product that may be safely consumed by a human or animal. Said food product may be in solid, semi-solid or liquid form and may comprise one or more nutrients, foods or nutritional supplements. For instance, the food product may additional comprise the following nutrients and micronutrients: a source of proteins, a source of lipids, a source of carbohydrates, vitamins and minerals. The composition may also contain anti-oxidants, stabilizers (when provided in solid form) or emulsifiers (when provided in liquid form).

The term "functional food product" as used herein, refers to a food product providing an additional health-promoting or disease-preventing function to the individual.

Food products and functional food products include for example cereal-based products, yogurts or other milk-derived products and bars.

The term "nutritional supplement", or "dietary supplement", as used herein, refers to a nutritional product that provides nutrients to an individual that may otherwise not be consumed in sufficient quantities by said individual. For instance, a nutritional supplement may include vitamins, minerals, fiber, fatty acids, or amino acids.

Supplements can for example be provided in the form of a pill, a tablet, a lozenger, a chewy capsule or tablet, a tablet or capsule, or a powder supplement that can for example be dissolved in water or sprinkled on food. Most preferred is a powder supplement that can be dissolved in liquid or sprinkled on food, most preferably dissolved in water. Such supplements typically provide the selected nutrients while not representing a significant portion of the overall nutritional needs of the subject. Typically they do not represent more than 0.1%, 1%, 5%, 10% or 20% of the daily energy need of the subject.

The term "dairy products", as used herein, refers to food products produced from animals such as cows, goats, sheep, yaks, horses, camels, and other mammals. Examples of dairy products are low-fat milk (e.g. 0.1%, 0.5% or 1.5% fat), fat-free milk, milk powder, whole milk, whole milk products, butter, buttermilk, buttermilk products, skim milk, skim milk products, high milk-fat products, condensed milk, crème fraiche, cheese, ice cream and confectionery products, probiotic drinks or probiotic yoghurt type drinks.

The term "pharmaceutical formulation" as used herein, refers to a composition comprising at least one pharmaceutically active agent, chemical substance or drug. The pharmaceutical formulation may be in solid or liquid form and can comprise at least one additional active agent, carrier, vehicle, excipient, or auxiliary agent identifiable by a person skilled in the art. The pharmaceutical formulation can be in the form of a tablet, capsule, granules, powder, liquid or syrup. The term "beverage product" as used herein, refers to a nutritional product in liquid or semi-liquid form that may be safely consumed by an individual.

The term "pet food product" as used herein refers to a nutritional product that is intended for consumption by pets. A pet, or companion animal, as referenced herein, is to be understood as an animal selected from dogs, cats, birds, fish, rodents such as mice, rats, and guinea pigs, rabbits, etc.

If the myo-inositol and probiotics are administration to the infant in-utero via the pregnant mother, they are preferably administered for at least 4, preferably at least 8, more preferably at least 12, more preferably at least 16, more preferably at least 20, more preferably at least 24, more preferably at least 28, even more preferably at least 36 weeks during pregnancy. As the nutritional requirements increase in the second and third trimester of pregnancy, it is preferred that administration comprises administration throughout the third trimester of pregnancy and most preferably throughout the second and third trimesters of pregnancy.

If the myo-inositol and probiotics are for administration to an infant after birth via the mother's breast milk, the myo-inositol may be administered partly or completely throughout the lactation or breastfeeding period of the mother.

The myo-inositol and probiotics may also be administered pre-pregnancy; this may positively impact the intrauterine environment. The myo-inositol and probiotics may for example be administered to a woman desiring to get pregnant, for example during at least 1, 2, 3 or 4 months preceding the pregnancy or desired pregnancy.

The myo-inositol and probiotics may be employed in any effective dose that provides a benefit with respect to improving the body composition of an infant, in particular with respect to preventing excess weight or lowering body weight in an infant and/or increasing the lean mass of an infant.

The term "dose" as used herein refers to a daily quantity that is administered to a subject.

The subject may be a pregnant mother or a mother, via whom the myo-inositol and probiotics are administered in-utero or via breast milk to the infant, or the subject may also be the infant to whom the myo-inositol and probiotics are administered directly.

It is well within the purview of the skilled person to determine an effective dose. The effective dose may depend on the form of administration in-utero e.g. via the pregnant mother, and if administration to the infant continues after birth, on the form of administration after birth e.g. via the mother's breast milk or directly to the infant.

With respect to improving body composition and increasing the % of lean body mass, an effective dose may be determined by air displacement plethysmography (ADP), total body water isotope dilution (TBW), total body electrical impedance analysis (TBEIA), dual X-ray absorptiometry (DXA), magnetic resonance imaging (MRI).

Any dose that resulting in an increase in the % of lean body mass of the infant may be considered an effective dose.

With respect to preventing excess weight an effective dose may be any dose that decreases the risk of an infant being or becoming overweight (having excess weight). Whether an infant is overweight (has excess weight) or is at risk of being overweight (having excess weight), may for example be determined from growth charts such as the standard growth charts issued by the WHO, or by calculating its z-score. An infant may be classified as having excess weight or being at risk of excess weight if they have a z-score of +2.

To determine an effective dose it may be necessary to compare infants supplemented in-utero, and optionally after birth, and infants not supplemented in-utero, and optionally after birth.

In an embodiment myo-inositol is administered in a dose from 0.2 to 5mg, 1.5 to 5mg, 2 to 4g, or 2g, and the probiotics are administered in a dose from 10⁵ to 10¹² colony forming units (cfu), or from 10⁷ to 10¹¹ cfu, in particular this dose is administered to a pregnant mother or a mother, via whom the myo-inositol and probiotics are administered in-utero or via breast milk to the infant.

The daily quantity or dose may be administered all at once or it may be spread out over several administrations throughout a day.

In an embodiment the dose is spread over 2 administrations, in a particular the dose is spread over 2 administrations, one in the morning and one in the evening.

In an embodiment the myo-inositol and probiotics are simultaneously, separately or sequentially administered with one or more of vitamin B2, vitamin B12, vitamin B6, and vitamin D. This may be particularly advantageous because said vitamins have a host of beneficial health effects associated with them, and in particular it is thought that maternal administration of one or more of these vitamins may affect the body composition of an infant, in particular the adiposity of an infant, or risk of obesity in an infant.

In a more particular embodiment vitamin B2 if used is administered in a dose of 0.14 to 14 mg, 1 to 2.5mg, 1.5 to 2mg, or 1.8mg; vitamin B6 if used is administered in a dose of 0.19 to 19 mg, or 2.6mg; vitamin B12 if used is administered in a dose of 0.26 to 26µg, or 5.2µg; and vitamin D if used is administered in a dose of 1.5 to 100 µg, 5 to 50 µg or 10µg ; in particular these doses are administered to a pregnant mother or a mother, via whom the myo-inositol and probiotics are administered in-utero or via breast milk to the infant.

The myo-inositol and probiotics may also be used in combination with other vitamins and minerals. For example, vitamins and minerals recommended by a governmental body, such as USRDA, for supplementation in pregnancy or during lactation e.g. calcium, magnesium, phosphorus, iron, zinc, copper, iodine, selenium, vitamin A or retinol activity equivalent (RAE) e.g. beta carotene or a mix of carotenoids, vitamin C, vitamin B1, niacin, folic acid, biotin, vitamin E.

The following micronutrients in the following amounts may be particularly useful when employed in combination with the myo-inositol and probiotics of the invention for the use herein described: 100 to 2500 mg calcium, 35 to 350 mg magnesium, 70 to 3500 mg phosphorus, 2.7 to 45 mg iron, 1.1 to 40 mg zinc, 0.1 to 10 mg copper, 22 to 1,100 µg iodine, 6 to 400 µg selenium, 77 to 3000 µg of vitamin A or retinol activity equivalent (RAE) for example in the form of beta carotene or a mix of carotenoids, 8.5 to 850 mg vitamin C, 0.14 to 14 mg Vitamin B1, 1.8 to 35 mg niacin, 60 to 1000 µg folic acid, 3 to 300 µg biotin, 1.9 to 109 µg Vitamin E; in particular these doses are administered to a pregnant mother or a mother, via whom the myo-inositol and probiotics are administered in-utero or via breast milk to the infant.

The combination of myo-inositol and one or more probiotic may also be used in combination with other ingredients commonly used in the form in which it is employed e.g. in the form of a composition e.g. a powdered nutritional supplement, or a food product. Non limiting examples of such ingredients include: other nutrients, for instance, selected from the group of lipids (optionally in addition to DHA and ARA), carbohydrates, and protein, micronutrients (in addition to those set out above), or pharmaceutically active agents; conventional food additives such as anti-oxidants, stabilizers, emulsifiers, acidulants, thickeners, buffers or agents for pH adjustment, chelating agents, colorants, excipients, flavor agents, osmotic agents, pharmaceutically acceptable carriers, preservatives, sugars, sweeteners, texturizers, emulsifiers, water and any combination thereof.

However, notwithstanding the above, a high fat intake during pregnancy and/or Maternal PUFA supplementation has been linked to an increased risk of higher adiposity in the offspring. Accordingly, in a particular embodiment the myo-inositol and one or more probiotic is not used in combination with a long chain polyunsaturated fatty acid and/or animal fats and/or vegetable fats.

In another aspect of the present invention there is provided a kit comprising myo-inositol and probiotics for sequential, separate or simultaneous administration, wherein the kit is for use to prevent excess weight and/or to increase the % lean mass of an infant. The kit may further comprise any optional ingredient with which myo-inositol and probiotics may be used.

In another aspect of the present invention there is provided myo-inositol and probiotics for use in the preparation of a composition for use in the manufacture of a composition for use to prevent excess weight and/or to increase the % lean mass of an infant.

In another aspect there is provided the non-therapeutic use of myo-inositol and probiotics for use to improve the body composition, more particularly to prevent excess weight and to increase the % lean body mass, in an infant, wherein said myo-inositol and probiotics are administered simultaneously, separately or sequentially to said infant in-utero and optionally also after birth.

Undernourished infants, or those born from undernourished or malnourished mothers or underweight mothers, may be more likely to suffer from a low % lean body mass. Accordingly myo-inositol and probiotics may be particularly beneficial for use in infants of these mothers to improve the body composition of said infants, in particularly to improve the % lean body mass.

A female human is considered as being underweight if she has a BMI of less than 18.5.

A female human is considered as being normal weight if she has a BMI of 18.5 to 25.

A female human may be considered as being undernourished or malnourished if they have a mid upper-arm circumference of less than 21-23 cm, more particularly less than 21cm.

Infants born from obese mothers or mothers who are overweight or have excess weight may be more likely to suffer from excess weight. Accordingly myo-inositol and probiotics may be particularly beneficial for use in infants of these mothers to improve the body composition of said infant, in particularly to prevent excess weight gain.

A female human is considered as being obese if she has a BMI of more than 30. A female human is considered as being overweight if she has a BMI of 25 to 30.

As would be clear to the skilled person, the invention disclosed herein has both therapeutic and non-therapeutic aspects.

There now follows a series of non-limiting examples that serve to illustrate the invention.

### Examples

### Example 1

80 Females virgin Goto Kakizaki (GK) rats of 4-5 weeks old with close body weight, were purchased from Charles River (France and USA), caged singly in a temperature-controlled room (22±1°C) with a 12-h light/dark cycle, and maintained on a AIN-93G growing rodent diet (Research diets, USA, **Table 1).**

After one week on acclimation, GK rats were divided into 4 groups (n=20) after a randomization based on AUC glucose value and body weight, and received their corresponding treatment for 10 weeks as below:
- Group Control: GK female rats ate *ad-libitum* a AIN-93G diet + a control gelatin **(Table 2).**
- Group Myo-inosito: GK female rats ate *ad-libitum* a AIN93-G diet supplemented with myo-inositol diet (1 g myo-inositol^{*} for 100 g de diet) + a control gelatin **(Table 2).**
- Group Probiotic: GK female rats ate *ad-libitum* a AIN93-G + a gelatin containing 10⁹ LPR CFU and 10⁹ B.lactis CFU **(Table2).**
- Group Mix : GK female ate *ad-libitum* a AIN93G diet supplemented with myo-inositol diet (1 g myo-inositol for 100g de diet) + a gelatin containing 10⁹ LPR CFU and 10⁹ B.lactis CFU **(Table 2).**
*Kirsh Pharma

At 15 weeks of age, females were housed with Wistar males (Charles Rivers, France) until a vaginal plug was observed, indicating day 0.5 of gestation. Female rats remained on their allocated experimental diet throughout gestation. At birth, only dams and their pups bearing at least 8 pups with minimum 3-4 males per litter, were included in the study. The number of pups in each litter was limited to 8, with preference to male pups. All dams were fed ad-libitum with a AIN-93G growing rodent diet (Research diets, USA, **Table 1)** during the lactation period. The pups remained with their mother until age of 21 days. During this period, they were allowed to suckle ad-libitum from dams. Then, they were housed two per cages and were fed an AIN93-G from d22 to d53. Body weight of the offspring was measured once per week, body composition was assessed at 21 days of age and all animals were sacrified at 100 days old.

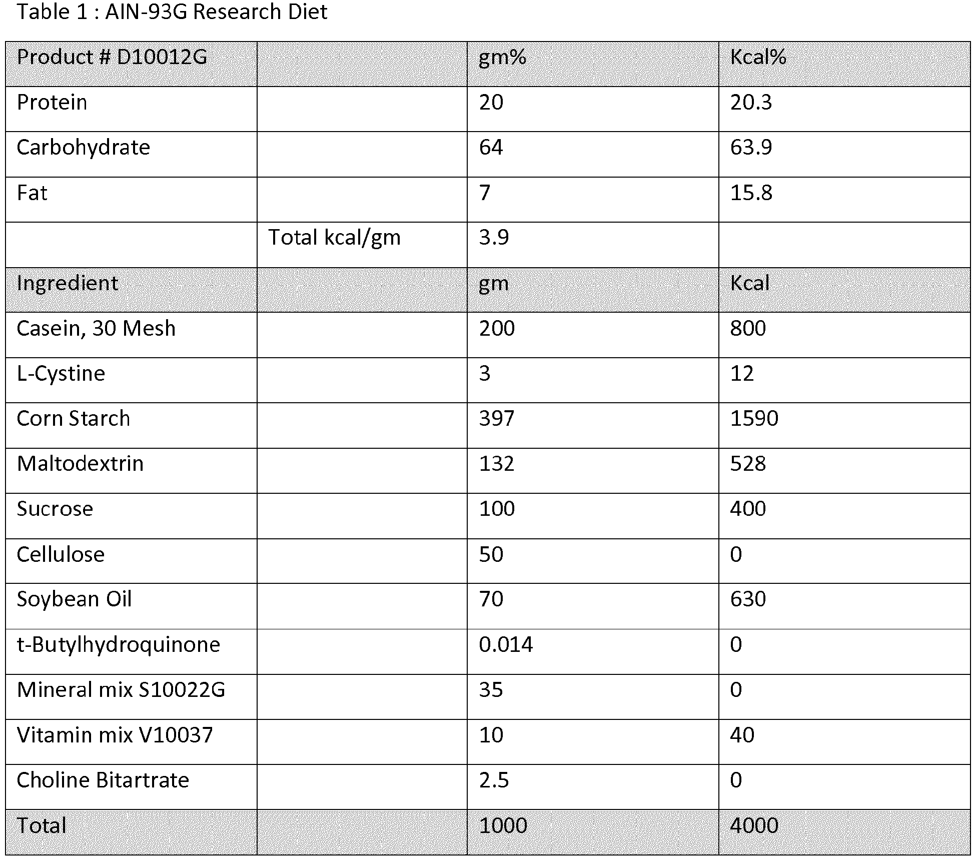

**Table 2b: Vitamin mix added to AIN-93G**

| **Ingredient** | **gm** | **Amount in 10gm** |
|---|---|---|
| Vitamin | 0.8 | 4000 IU |
| A, Acetate (500,000 IU/gm) | | |
| Vitamin D3 (100,000 IU/gm) | 1 | 1,000 IU |
| Vitamin E Acetate (500 IU/gm) | 15 | 75 IU |
| Phylloquinone | 0.075 | 0.75 mg |
| Biotin, 1.0% | 2 | 0.2 mg |
| Cyanocobalamin, 0.1% | 2.5 | 25 µg |
| Folic Acid | 0.2 | 2 mg |
| Nicotinic Acid | 3 | 30 mg |
| Calcium Pantothenate | 1.6 | 16 mg |
| Pyridoxine---HCl | 0.7 | 7 mg |
| Riboflavin | 0.6 | 6 mg |
| Thiamin HCl | 0.6 | 6 mg |
| Sucrose | 971.925 | |
| **TOTAL** | **1000** | |

**Table 2 : Composition of the experimental Gelatin**

| | Treatment | Control |
|---|---|---|
| Gelatin (g) | 12 | 12 |
| Sacharine (mg) | 0.06 | 0.06 |
| Probiotic (g)* | 7.7 | 0 |
| Maltodextrin mix (g) | 0 | 6.7 |
| TS + Water (ml) | 80.2 | 81.2 |
| Total (ml) | 99.96 | 99.96 |

| | | |
|---|---|---|
| *Premix containing 2.53 E+10 cfu/g Lactobacillts rhamnosus (NCC4007) and 1.58E+10 cfu/g of Bifido Lactis (NCC2818). | | |

### Measurements and analysis

### Physiological measurements and sample collections:

- The body weight of the offspring was measured 1-2 times / week throughout the study.
- Lean body mass was measured at 21 days of age by using nuclear magnetic resonance (EchoMRI TM 2004, Echo Medical Systems, Houston, USA). From these data collected it was possible to calculate the %lean mass as following :
   - % lean mass : lean mass (g) / body weight

Sacrifice: offspring were sacrificed under under anesthesia (isofluarane) after 6 hours of day deprivation (from 7.30 am to 13.30 pm). Blood was collected in EDTA tubes and plasma FFA was measured by Cobas. (Cobas C111, Roche).
- Insulin pancreatic measurement: Pancreas was weighted during the sacrifice and preserved in an acid-ethanol-H2O solution until insulin extraction. For determination of pancreatic insulin, pancreas was homogenized with an acid-ethanol (solution v/v: 75% ethanol, 1.5% of 37% HCI and 23.5% distilled water) and incubated at -20°C overnight. The insulin content in the supernatant was measured by an ELISA method using kits from Crystal Chem. Inc (IL, USA).

Results: The mean value for each measurement is shown in table 3

**Table 3**

| | Control | Myo-inositol | Probiotics | Mix (myo-inositol+probiotics) |
|---|---|---|---|---|
| % lean mass at 21 days old | 82.8 | 81.8 | 82.1 | 83.2 |
| lean mass in g at 21 days old | 36.2 | 39.3 | 38.2 | 34.6 |
| Body weight in g at 21 days old | 44.3 | 48.1 | 46.4 | 43.9 |
| µg insulin in total pancreas | 84 | 89 | 90 | 107 |
| µg insulin pancreas/ g pancreas | 68 | 76 | 75 | 89 |
| FFA plasmatic (mmol/l) | 0.43 | 0.41 | 0.43 | 0.40 |

### Example 4

An example composition comprising a combination of myo-inositol and probiotics (Lactobacillus rhamnosus GG1 and Bifidobacterium lactis BB122) further combined within vitamin B2 and vitamins B6, B12 and D, is set out in table 4.

The composition in table 4 is for a nutritional supplement in a powder form, intended to be sprinkled on food and administered to a pregnant mother or mother via whom the ingredients, in particular myo-inositol and probiotics, are administered in-utero or via breast milk to an infant.

**Table 4 : Composition**

| Ingredient | Amount per daily dose |
|---|---|
| Myo-inositol | 4g |
| Vitamin D | 10 µg |
| Vitamin B6 | 2.6 mg |
| Vitamin B12 | 5.2 µg |
| Vitamin B2 | 1.8 mg |
| Zinc | 10 mg |
| β-carotene | 720 µg |
| Folic acid | 400 µg |
| Iron | 12 µg |
| Calcium | 150 µg |
| Iodine | 150 µg |
| Lactobacillus rhamnosus GG1) | 1x109 cfu |
| Bifidobacterium lactis BB122) | 1x109 cfu |

Strain deposited as CGMCC 1.3724
Strain deposited as CNCM I-3446

The composition may, for example, be provided as a kit of parts comprising in one sachet the probiotics as a powder and in a second sachet all other ingredients.

### Example 3

A milk powder drink for pregnant women to be reconstituted in water is provided consisting of 30g of milk powder per serving admixed with the ingredients listed in Table 1, in half of the amounts mentioned in Table 1. The composition is to be administered to a pregnant woman twice per day during at least the third trimester of pregnancy.

## Claims

1. Myo-inositol and probiotics for use in the prevention of excess body weight of an infant, wherein said myo-inositol and probiotics are administered simultaneously, separately or sequentially to said infant in-utero and optionally also after birth wherein, said probiotics comprises a combination of Lactobacillus and Bifidobacterium, and wherein the Lactobacillus is the Lactobacillus rhamnosus GG strain available under the deposit number CGMCC 1.3724, and the Bifidobacterium is the Bifidobacterium lactis BB12 strain deposited as CNCM 1-3446.

2. Myo-inositol and probiotics for use according to claim 1, wherein the myo-inositol and probiotics are administered in-utero via the pregnant mother.

3. Myo-inositol and probiotics for use according to claim 1 or 2, wherein when said myo-inositol and probiotics are administered also after birth they are administered via the mother's breast milk.

4. Myo-inositol and probiotics for use according to claim 1, 2 or 3 wherein when said myo-inositol and probiotics are administered also after birth they are administered up to until the infant is 12 months of age.

5. Myo-inositol and probiotics for use according to claim 1, 2, 3 or 4, wherein when said myo-inositol and probiotics are administered also after birth they are administered directly to the infant.

6. Myo-inositol and probiotics for use according to claim 1, 2, 3, 4, or 5, wherein said myoinositol and probiotics are administered simultaneously, separately or sequentially with one or more of vitamin B2, vitamin B12, vitamin B6, and vitamin D.

7. Myo-inositol and probiotics for use according to claim 1, 2, 3, 4, 5, or 6 wherein said myoinositol is administered in a daily dose equating to 0.2 to 5 mg of myo-inositol, and
said probiotics are administered in a daily dose equating to 10₅ to 10₁₂ cfu, and wherein preferably the dose is administered to a pregnant mother or a mother, via whom the myo-inositol and probiotics are administered in-utero or via breast milk to the infant..

8. Myo-inositol and probiotics for use according to claim 1, 2, 3, 4, 5, 6, or 7, wherein said myoinositol and probiotics are administered simultaneously in the form of a composition and wherein said composition optionally also comprises one or more of vitamin B2, vitamin B12, vitamin B6, and vitamin D, and wherein said vitamin B2, vitamin B6, vitamin B12, and/or vitamin D are preferably administered in the following daily doses; 0.14 to 14 mg of vitamin B2, 0.19 to 19 mg of vitamin B6, 0.26 to 26 µg of vitamin B12, 1.5 to 100 µg of vitamin D and wherein preferably these doses are administered to a pregnant mother or a mother, via whom the myo-inositol and probiotics are administered in-utero or via breast milk to the infant.

9. Myo-inositol and probiotics for use according to claim 8, wherein when said composition is administrated in-utero via the mother or after birth via the mother's breast milk said composition is a composition suitable for maternal administration, and wherein when said composition is administered after birth directly to the infant said composition is a composition suitable for consumption by an infant.

## Patentansprüche

1. myo-Inosit und Probiotika zum Gebrauch bei der Vorbeugung von überschüssigem Körpergewicht eines Säuglings, wobei das myo-Inosit und die Probiotika dem Säugling gleichzeitig, separat oder sequenziell im Uterus und wahlweise auch nach der Geburt verabreicht werden, wobei die Probiotika eine Kombination von Lactobacillus und Bifidobacterium umfassen und wobei der Lactobacillus der Stamm Lactobacillus rhamnosus GG ist, der unter der Depotnummer CGMCC 1.3724 erhältlich ist, und das Bifidobacterium der Stamm Bifidobacterium lactis BB12, deponiert als CNCM I-3446, ist.

2. myo-Inosit und Probiotika zum Gebrauch nach Anspruch 1, wobei das myo-Inosit und die Probiotika über die schwangere Mutter im Uterus verabreicht werden.

3. myo-Inosit und Probiotika zum Gebrauch nach Anspruch 1 oder 2, wobei, wenn das myo-Inosit und die Probiotika auch nach der Geburt verabreicht werden, diese über die Muttermilch der Mutter verabreicht werden.

4. myo-Inosit und Probiotika zum Gebrauch nach Anspruch 1, 2 oder 3, wobei, wenn das myo-Inosit und die Probiotika auch nach der Geburt verabreicht werden, diese verabreicht werden, bis der Säugling 12 Monate alt ist.

5. myo-Inosit und Probiotika zum Gebrauch nach Anspruch 1, 2, 3 oder 4, wobei, wenn das myo-Inosit und die Probiotika auch nach der Geburt verabreicht werden, diese dem Säugling direkt verabreicht werden.

6. myo-Inosit und Probiotika zum Gebrauch nach Anspruch 1, 2, 3, 4 oder 5, wobei das myo-Inosit und die Probiotika gleichzeitig, separat oder sequenziell mit einem oder mehreren von Vitamin B2, Vitamin B12, Vitamin B6 und Vitamin D verabreicht werden.

7. myo-Inosit und Probiotika zum Gebrauch nach Anspruch 1, 2, 3, 4, 5 oder 6, wobei das myo-Inosit in einer Tagesdosis gleich 0,2 bis 5 mg myo-Inosit verabreicht wird und die Probiotika in einer Tagesdosis von gleich 10₅ bis 10₁₂ KBE verabreicht werden und wobei vorzugsweise die Dosis einer schwangeren Mutter oder einer Mutter verabreicht wird, über die das myo-Inosit und die Probiotika im Uterus oder über die Muttermilch an den Säugling verabreicht werden.

8. myo-Inosit und Probiotika zum Gebrauch nach Anspruch 1, 2, 3, 4, 5, 6 oder 7, wobei das myo-Inosit und die Probiotika gleichzeitig in der Form einer Zusammensetzung verabreicht werden und wobei die Zusammensetzung wahlweise auch ein oder mehrere von Vitamin B2, Vitamin B12, Vitamin B6 und Vitamin D umfasst und wobei das Vitamin B2, Vitamin B6, Vitamin B12 und/oder Vitamin D vorzugsweise in den folgenden Tagesdosen verabreicht werden; 0,14 bis 14 mg Vitamin B2, 0,19 bis 19 mg Vitamin B6, 0,26 bis 26 µg Vitamin B12, 1,5 bis 100 µg Vitamin D, und wobei vorzugsweise diese Dosen einer schwangeren Mutter oder einer Mutter verabreicht werden, über die das myo-Inosit und die Probiotika im Uterus oder über die Muttermilch an den Säugling verabreicht werden.

9. myo-Inosit und Probiotika zum Gebrauch nach Anspruch 8, wobei, wenn die Zusammensetzung im Uterus über die Mutter oder nach der Geburt über die Muttermilch der Mutter verabreicht wird, die Zusammensetzung eine Zusammensetzung ist, die zur mütterlichen Verabreichung geeignet ist, und wobei, wenn die Zusammensetzung nach der Geburt direkt an den Säugling verabreicht wird, die Zusammensetzung eine Zusammensetzung ist, die zum Verzehr durch einen Säugling geeignet ist.

## Revendications

1. Myo-inositol et probiotiques utilisables dans la prévention de l'excès de poids corporel d'un nourrisson, lesdits myo-inositol et probiotiques étant administrés simultanément, séparément ou séquentiellement audit nourrisson in-utero et éventuellement également après la naissance, lesdits probiotiques comprenant une combinaison de Lactobacillus et de Bifidobacterium, et le Lactobacillus étant la souche Lactobacillus rhamnosus GG disponible sous le numéro de dépôt CGMCC 1.3724, et le Bifidobacterium étant la souche Bifidobacterium lactis BB12 déposée en tant que CNCM I-3446.

2. Myo-inositol et probiotiques utilisables selon la revendication 1, le myo-inositol et les probiotiques étant administrés in-utero par l'intermédiaire de la mère enceinte.

3. Myo-inositol et probiotiques utilisables selon la revendication 1 ou 2, selon lesquelles, lorsque lesdits myo-inositol et probiotiques sont administrés également après sa naissance, ils sont administrés via le lait maternel.

4. Myo-inositol et probiotiques utilisables selon la revendication 1, 2 ou 3 selon lesquelles, lorsque lesdits myo-inositol et probiotiques sont administrés également après la naissance, ils sont administrés jusqu'à ce que le nourrisson soit âgé de 12 mois.

5. Myo-inositol et probiotiques utilisables selon la revendication 1, 2, 3 ou 4, selon lesquelles, lorsque lesdits myo-inositol et probiotiques sont administrés également après la naissance, ils sont administrés directement au nourrisson.

6. Myo-inositol et probiotiques utilisables selon la revendication 1, 2, 3, 4 ou 5, lesdits myo-inositol et probiotiques étant administrés simultanément, séparément ou séquentiellement avec une ou plusieurs parmi la vitamine B2, la vitamine B12, la vitamine B6, et la vitamine D.

7. Myo inositol et probiotiques utilisables selon la revendication 1, 2, 3, 4, 5 ou 6, ledit myo-inositol étant administré à une dose quotidienne 0,2 à 5 mg de myo-inositol, et lesdits probiotiques étant administrés à une dose quotidienne égale à 10₅ à 10₁₂ ufc, et de préférence la dose étant administrée à une femme enceinte ou une mère, par l'intermédiaire de laquelle le myo-inositol et les probiotiques sont administrés au nourrisson in-utero ou via le lait maternel.

8. Myo-inositol et probiotiques utilisables selon la revendication 1, 2, 3, 4, 5, 6 ou 7, lesdits myo-inositol et probiotiques étant administrés simultanément sous la forme d'une composition et ladite composition comprenant en outre facultativement une ou plusieurs parmi la vitamine B2, la vitamine B12, la vitamine B6, et la vitamine D, et lesdites vitamine B2, vitamine B6, vitamine B12, et/ou vitamine D étant administrées de préférence aux doses quotidiennes suivantes ; 0,14 À 14 mg de vitamine B2, 0,19 à 19 mg de vitamine B6, 0,26 à 26 µg de vitamine B12, 1,5 à 100 µg de vitamine D, et ces doses étant de préférence administrées à une femme enceinte ou à une mère, par l'intermédiaire de laquelle le myo-inositol et les probiotiques sont administrés au nourrisson in-utero ou via le lait maternel.

9. Myo-inositol et probiotiques utilisables selon la revendication 8, selon laquelle lorsque ladite composition est administrée in-utero par l'intermédiaire de la mère ou après la naissance via le lait maternel, ladite composition est une composition appropriée pour une administration maternelle, et selon laquelle, lorsque ladite composition est administrée après la naissance directement au nourrisson, ladite composition est une composition appropriée pour la consommation par un nourrisson.
